(19) **Europäisches Patentamt / European Patent Office / Office européen des brevets**

(11) **EP 4 237 140 B1**

(12) **FASCICULE DE BREVET EUROPEEN**

(45) Date de publication et mention de la délivrance du brevet:
**19.03.2025 Bulletin 2025/12**

(21) Numéro de dépôt: **21810673.0**

(22) Date de dépôt: **29.10.2021**

(51) Classification Internationale des Brevets (IPC):
**B01J 21/06** (2006.01)    **B01J 12/02** (2006.01)
**B01J 23/83** (2006.01)    **C07C 1/02** (2006.01)

(52) Classification Coopérative des Brevets (CPC):
(C-Sets disponibles)
**B01J 21/066; B01J 19/088; B01J 23/83; C07C 1/12; C10L 3/08;** B01J 2219/0892; B01J 2219/0896; C07C 2521/06; C07C 2523/10; C07C 2523/83; C10L 2290/38 (Cont.)

(86) Numéro de dépôt international:
**PCT/FR2021/051906**

(87) Numéro de publication internationale:
**WO 2022/090671 (05.05.2022 Gazette 2022/18)**

(54) **SYSTÈME CATALYTIQUE MIXTE POUR LA CONVERSION DU CO2 ET/OU DU CO DANS UN PROCÉDÉ HYBRIDE PLASMA FROID-CATALYSE**

GEMISCHTES KATALYTISCHES SYSTEM ZUR UMWANDLUNG VON CO2 UND/ODER CO IN EINEM HYBRIDEN KALTPLASMAKATALYSEVERFAHREN

MIXED CATALYTIC SYSTEM FOR THE CONVERSION OF CO2 AND/OR OF CO IN A COLD PLASMA-CATALYSIS HYBRID PROCESS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.10.2020 FR 2011113**

(43) Date de publication de la demande:
**06.09.2023 Bulletin 2023/36**

(73) Titulaires:
• **Paris Sciences et Lettres**
  **75006 Paris (FR)**
• **CENTRE NATIONAL DE LA RECHERCHE SCIENTIFIQUE (CNRS)**
  **75016 Paris (FR)**
• **SORBONNE UNIVERSITE**
  **75006 Paris (FR)**
• **Ecole Nationale Superieure de Chimie Paris**
  **75231 Paris Cedex 05 (FR)**

(72) Inventeurs:
• **GALVEZ-PARRUCA, Maria Elena**
  **93500 PANTIN (FR)**
• **MIKHAIL, Maria**
  **75013 PARIS (FR)**
• **OGNIER, Stéphanie**
  **75013 PARIS (FR)**
• **DA COSTA, Patrick**
  **93500 PANTIN (FR)**
• **CAVADIAS, Simeon**
  **75015 PARIS (FR)**
• **TATOULIAN, Michael**
  **75013 PARIS (FR)**

(74) Mandataire: **Alatis**
  **3, rue Paul Escudier**
  **75009 Paris (FR)**

(56) Documents cités:
EP-A1- 3 050 865        WO-A1-2013/156550
WO-A1-2020/128009

- **YAO LU ET AL: "Synthesis Gas Production via Dry Reforming of Methane over Manganese Promoted Nickel/Cerium-Zirconium Oxide Catalyst", INDUSTRIAL & ENGINEERING CHEMISTRY RESEARCH, vol. 57, no. 49, 15 November 2018 (2018-11-15), pages 16645 - 16656, XP055879284, ISSN: 0888-5885, DOI: 10.1021/acs.iecr.8b04183**
- **FAKEEHA ANIS H. ET AL: "Yttria Modified ZrO 2 Supported Ni Catalysts for CO 2 Reforming of Methane: The Role of Ce Promoter", ACS OMEGA, vol. 6, no. 2, 7 January 2021 (2021-01-07), US, pages 1280 - 1288, XP055879291, ISSN: 2470-1343, Retrieved from the Internet <URL:http://pubs.acs.org/doi/pdf/10.1021/acsomega.0c04731> DOI: 10.1021/acsomega.0c04731**

(52) Classification Coopérative des Brevets (CPC):
(Cont.)

C-Sets
**C07C 1/12, C07C 9/04**

**Description**

**DOMAINE DE L'INVENTION**

La présente invention concerne un nouveau système catalytique susceptible d'être activé par un plasma froid, notamment généré par « décharge à barrière diélectrique », dit plasma DBD, et le procédé de préparation dudit système catalytique. L'invention a également trait à un procédé de conversion du $CO_2$ et/ou du CO mettant en œuvre ledit système catalytique, par exemple pour produire des hydrocarbures, en particulier du méthane, des alcools, du monoxyde de carbone ou encore de l'acide formique.

**ETAT DE LA TECHNIQUE**

La réduction des émissions de $CO_2$ dans l'atmosphère représente actuellement un besoin important, en raison de l'augmentation exponentielle de ces émissions de $CO_2$ au cours de la dernière décennie, ayant entraîné d'importants dommages environnementaux tels que le réchauffement climatique ou l'acidification de l'eau de mer. Par conséquent, les technologies qui impliquent la conversion du $CO_2$ en carburants offrent des avantages précieux pour réduire une quantité importante de $CO_2$ et stocker cette énergie renouvelable. A cet effet, le dioxyde de carbone peut être transformé sous la forme d'un élément valorisable, typiquement du méthane.

Ainsi, parmi les différentes réactions d'hydrogénation du $CO_2$, la plus connue est la réaction de Sabatier (1) qui permet de convertir le $CO_2$ en méthane. Le méthane représente un combustible neutre en carbone et permet le stockage d'électricité renouvelable en dehors des heures de pointe (concept de « power-to-gas »).

$$CO_2(g) + 4H_2(g) \rightarrow CH_4(g) + 2H_2O(g), \Delta H°25°C = -165,3 \text{ kJ/mol} \qquad (1)$$

La réaction de Sabatier est exothermique et thermodynamiquement favorable à basse température. Cependant, elle est sérieusement entravée par sa cinétique de réaction lente en raison de la grande stabilité du $CO_2$. Ainsi, l'utilisation de catalyseurs s'avère primordiale pour obtenir un taux de conversion satisfaisant.

Ocampo et al. (1) a décrit l'utilisation de solutions solides d'oxyde mixte de cérium et de zirconium pour la préparation de catalyseurs au nickel pour la méthanation du $CO_2$. Les oxydes mixtes $CeO_2$-$ZrO_2$ présentent de bonnes caractéristiques physico-chimiques, telles qu'une bonne capacité de stockage de l'oxygène et une bonne mobilité, une basicité de surface accrue favorisant l'adsorption du $CO_2$, ainsi qu'une stabilité thermique adéquate.

Les plasmas froids, appelés de manière équivalente « plasmas non thermiques », notamment les plasmas générés par décharge à barrière diélectrique (DBD), peuvent être utilisés en association avec un catalyseur pour améliorer les performances de la méthanation du $CO_2$ (2)-(9). Un tel procédé hybride plasma-catalyse présente plusieurs avantages par rapport à la catalyse conventionnelle, car il fonctionne à pression atmosphérique. Ce procédé hybride peut être réalisé à basse température (e.g. 180°C-240°C) et permet d'obtenir une meilleure sélectivité en méthane (> 95%), sans aucune réaction secondaire. Toutefois, il reste très coûteux en énergie. Le coût de l'énergie est défini comme la quantité d'énergie consommée par le processus par molécule convertie, exprimé souvent en kJ ou eV par molécule.

Les inventeurs ont précédemment démontré l'association efficace d'un catalyseur à base d'oxyde mixte de cérium et de zirconium, imprégné avec du nickel avec un plasma froid tel qu'un plasma DBD pour effectuer la réaction de Sabatier à basse température (e.g. inférieure à 250°C) (10). Toutefois, avec ce système catalytique, la consommation énergétique reste importante pour obtenir un taux de conversion du $CO_2$ satisfaisant.

Il existe donc un besoin pour un catalyseur efficace pour la réaction de conversion d'un gaz comprenant du $CO_2$ et/ou du monoxyde de carbone (CO), à l'aide d'un procédé hybride plasma froid -catalyse, ledit catalyseur permettant une réduction de la consommation d'énergie dans la réaction et/ou une amélioration des performances catalytiques, à savoir le taux de conversion du gaz, la sélectivité et la stabilité catalytique, afin d'obtenir par exemple des hydrocarbures ou des alcools. WO 2020/128009 divulgue un réacteur pour la conversion du dioxyde de carbone.

**RESUME DE L'INVENTION**

Un but de l'invention est donc de proposer un nouveau catalyseur, encore appelé système catalytique, permettant de convertir le $CO_2$ et/ ou le CO en carburant, incluant les hydrocarbures, les alcools, l'acide formique, le monoxyde de carbone (si la réaction est réalisée à partir de $CO_2$) et leurs mélanges, avec une consommation réduite en énergie et un taux de conversion du $CO_2$ et/ ou du CO amélioré par rapport à ceux décrits dans l'art antérieur.

Un premier objet de la présente invention porte sur un système catalytique comprenant :

- un support comprenant du cérium et/ou du zirconium,
- du nickel, et

- un promoteur choisi parmi le gadolinium et les mélanges gadolinium et yttrium.

Un second objet de la présente invention concerne un procédé de préparation d'un système catalytique tel que défini ci-dessus par mise en contact du support ou un précurseur de celui-ci avec un précurseur de nickel et un précurseur du promoteur.

Un troisième objet de la présente invention porte sur un procédé de conversion d'un gaz comprenant du $CO_2$ et/ou du CO en présence d'un système catalytique tel que défini ci-dessus et d'un plasma froid.

## DESCRIPTION DES FIGURES

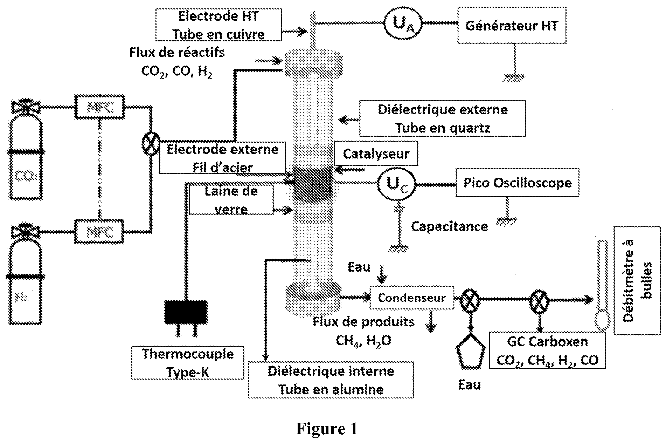

La figure 1 illustre le dispositif expérimental utilisé dans un exemple d'hydrogénation du $CO_2$, la méthanation, par un procédé plasma DBD + catalyse selon la présente invention.

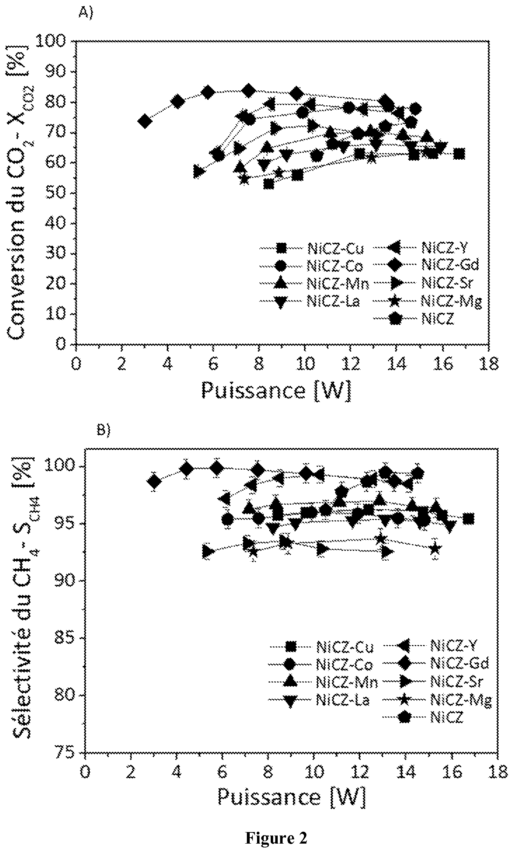

La figure 2 illustre l'activité catalytique sous plasma DBD de systèmes catalytiques selon l'invention pour la méthanation du $CO_2$ : A) taux de conversion du $CO_2$ obtenu en fonction de la consommation énergétique (puissance) avec un système catalytique témoin (NiCZ) ou un système catalytique selon l'invention ; B) sélectivité du méthane ($CH_4$) obtenue en fonction de la consommation énergétique (puissance) avec un système catalytique témoin (NCZ) ou un système catalytique selon l'invention.

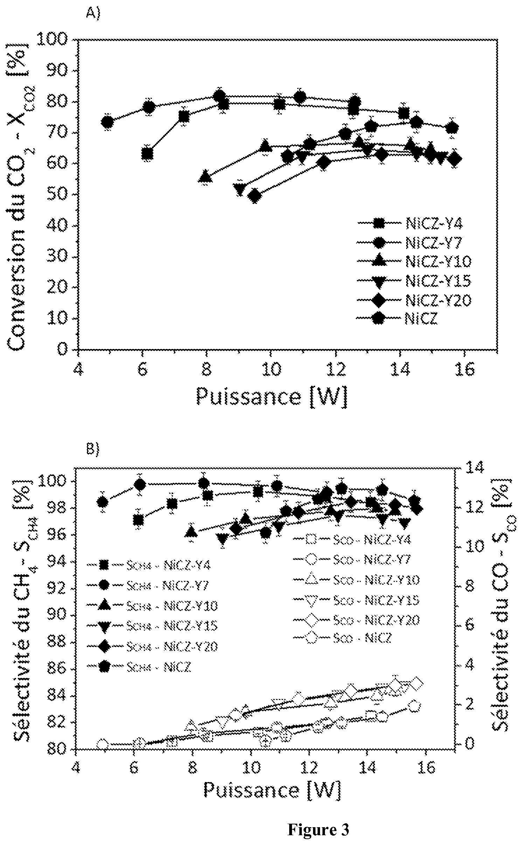

La figure 3 illustre l'activité catalytique sous plasma DBD de systèmes catalytiques pour la méthanation du $CO_2$ : A) taux de conversion du $CO_2$ obtenue en fonction de la consommation énergétique (puissance) avec un système catalytique témoin (NiCZ) ou un système catalytique avec de l'yttrium à différentes teneurs massiques (pour illustration seulement) ; B) sélectivité du méthane ($CH_4$) obtenue par rapport au CO en fonction de la consommation énergétique (puissance) avec un système catalytique témoin ou un système catalytique avec de l'yttrium à différentes teneurs massiques.

## DESCRIPTION DETAILLEE DE L'INVENTION

### *Le système catalytique*

La présente invention concerne un système catalytique comprenant :

- un support comprenant du cérium et/ou du zirconium,
- du nickel, et
- un promoteur choisi parmi le gadolinium et les mélanges gadolinium et yttrium.

Dans le cadre de la présente invention, les termes « système catalytique » et « catalyseur » sont interchangeables.

Le support selon l'invention est notamment choisi de par sa capacité à adsorber le $CO_2$ et/ou le CO à sa surface et sa stabilité thermique. Par « cérium » et « zirconium », on entend au sens de la présente invention du cérium et du zirconium sous tous leurs états d'oxydation. Préférablement, le terme « cérium » utilisé pour définir le support désigne de l'oxyde de cérium $CeO_2$ et le terme « zirconium » désigne de l'oxyde de zirconium $ZrO_2$.

Le support pourra ainsi comprendre, notamment sera constitué, de cérium, de zirconium ou d'un mélange de cérium et de zirconium. Avantageusement, le support comprend, notamment est constitué de cérium, éventuellement en combinaison avec du zirconium. Plus préférablement, le support du système catalytique comprend, notamment est constitué par, un mélange de cérium et de zirconium.

De manière avantageuse, le support pourra ainsi comprendre, notamment sera constitué, d'un oxyde de cérium, d'un oxyde de zirconium ou d'un oxyde mixte de cérium et de zirconium. Avantageusement, le support comprend, notamment est constitué, d'oxyde de cérium, éventuellement en combinaison avec de l'oxyde de zirconium. Plus préférablement, le support du système catalytique comprend, notamment est constitué par, un oxyde mixte de cérium et de zirconium (abrégé $CeO_2$-$ZrO_2$ ou oxyde mixte CeZr ou CZ).

Lorsque le support est un mélange de cérium et de zirconium, notamment un oxyde mixte CeZr, le ratio molaire cérium/zirconium (Ce/Zr) est avantageusement compris dans une gamme allant de 90/10 à 40/60, préférablement de 80/20 à 50/50, notamment 70/30 à 50/50. De préférence, le ratio cérium/zirconium est d'environ 60/40. Le ratio Ce/Zr va notamment moduler les performances catalytiques du système. En effet, l'adsorption des réactifs à la surface du support est généralement favorisée par la présence de défauts inhérents présents sur ladite surface, principalement dus à un manque d'oxygène. L'ajout d'ions Zr4+ dans le réseau de l'oxyde de cérium lors de la formation de l'oxyde mixte CeZr permet un nombre plus élevé de défauts et favorise la mobilité de l'oxygène à la surface du support conduisant ainsi à des performances catalytiques accrues.

Par « environ », on entend dans la présente description que la valeur concernée peut être inférieure ou supérieure de 10%, notamment de 5%, en particulier de 1%, à la valeur indiquée. Le nickel correspond à la phase active du système catalytique. Par « nickel », on entend au sens de la présente invention du nickel sous tous ses états d'oxydation. Il peut ainsi s'agir de nickel sous son état d'oxydation 0, c'est-à-dire sous forme métallique, sous son état d'oxydation II (e.g. sous forme d'oxyde de nickel (II) (NiO)) ou encore sous son état d'oxydation III (e.g. sous forme d'oxyde de nickel (III) ($Ni_2O_3$)). La teneur massique en nickel dans le système catalytique va avantageusement de 3% à 30% en poids par rapport au poids du support, de préférence de 5% à 20%, encore plus préférablement de 7% à 17%. De manière particulièrement avantageuse, le système catalytique comprend environ 15% en poids de nickel par rapport au poids du support.

Le système catalytique selon l'invention permet d'améliorer la performance énergétique lors de la conversion, notamment l'hydrogénation, du gaz comprenant du $CO_2$ et/ou du CO en présence d'un plasma non-thermique avec une diminution de la puissance consommée, et de préférence tout en améliorant les performances catalytiques de la réaction, c'est-à-dire le taux de conversion du $CO_2$ et/ou du CO et/ou la sélectivité vers le produit désiré.

A cet effet, le système catalytique comprend un promoteur qui agit comme un dopant et qui va notamment modifier les propriétés conductrices du système catalytique. Le promoteur selon l'invention possède avantageusement des propriétés physico-chimiques de surfaces adéquates pour aider à fixer le $CO_2$ et/ou le CO et une constante diélectrique qui permet d'améliorer la conductivité du système catalytique résultant. Par « propriétés physico-chimiques de surfaces adéquates », on entend par exemple un grand nombre de défauts de surface, une basicité appropriée, une lacune en oxygène et/ou un rayon ionique adéquat pour l'incorporation dans le réseau cristallin de l'oxyde de cérium et la favorisation des cycles rédox.

Ainsi, dans le cadre de la présente invention, le promoteur est choisi parmi le gadolinium et les mélanges gadolinium et yttrium. Dans le cadre de la présente invention, le promoteur se trouve sous n'importe lequel de ses états d'oxydation et notamment sous forme métallique ou sous forme d'oxyde.

Le promoteur est avantageusement le gadolinium.

La teneur massique en promoteur dans le système catalytique varie de 0,1% à 20% en poids par rapport au poids du support, par exemple de 0,2 à 20% en poids, notamment de 0,5% à 15% en poids, de préférence de 1% à 10% en poids, notamment de 2% à 8% en poids, encore plus préférablement de 4 à 7% en poids.

La quantité de nickel et/ou de promoteur peut être ajustée en fonction de la nature du promoteur utilisée et/ou du type de réaction de conversion mise en œuvre, c'est-à-dire selon le type de produit généré par la réaction de conversion, notamment d'hydrogénation.

Ainsi, selon un mode de réalisation, lorsque le promoteur est le gadolinium, sa teneur massique dans le système catalytique est préférablement d'environ 4% en poids par rapport au poids du support.

Le système catalytique est avantageusement sous forme de poudre. De préférence, les grains formant la poudre ont une taille moyenne comprise entre 1 $\mu$m et 1 mm, par exemple entre 100 $\mu$m et 1 cm, notamment entre 200$\mu$m et 800$\mu$m, préférentiellement environ 500 $\mu$m. La taille des grains peut notamment être adapté en fonction de l'échelle de production utilisée. Le système catalytique peut également se trouver sous forme de billes (notamment par compression de la poudre dans un moule) ayant une taille moyenne comprise entre 1 mm et 5 cm. La détermination de la taille moyenne des grains et des billes peut être effectuée par granulométrie laser.

Avantageusement, le support le nickel et le promoteur forment un mélange homogène. On entend par là que le nickel et le promoteur sont répartis uniformément dans la totalité du volume du catalyseur.

### *Le procédé de préparation du système catalytique*

La présente invention porte également sur un procédé de préparation d'un système catalytique tel que défini ci-dessus comprenant une étape de mise en contact du support ou d'un précurseur de celui-ci avec un précurseur de nickel et un précurseur du promoteur, éventuellement suivie d'une étape de calcination, qui sera éventuellement suivi d'une étape de réduction.

L'étape de mise en contact du support avec un précurseur de nickel et un précurseur du promoteur va permettre de former un solide comprenant le support, le nickel et le promoteur. De préférence, ce solide sera ensuite calciné. Une telle étape de calcination peut être effectuée par exemple à une température comprise entre 300 et 600°C. Cette étape de calcination peut être réalisée par exemple pendant 3h ou plus, notamment entre 3h et 6h. L'étape de calcination peut également être réalisée *in situ* dans le dispositif de plasma non thermique, par exemple pendant une durée de 3h ou plus, de préférence entre 3h et 5h, à une température comprise entre 300°C et 600°C. L'étape de calcination conduit à l'oxydation des différents composants du système catalytique (support, nickel et promoteur).

L'étape de calcination pourra éventuellement être suivie d'une étape de réduction de sorte à changer l'état d'oxydation du nickel et du promoteur (pour avoir du nickel et du promoteur métallique), et éventuellement celui du support. La réduction peut être totale ou partielle, c'est-à-dire que seule une partie des composants du système catalytique peut être réduite. L'étape de réduction peut être réalisée *in situ* dans le dispositif de plasma non thermique sous hydrogène.

Plusieurs (par exemple 2) séquences comprenant une étape de mise en contact et une étape de calcination peuvent être réalisées successivement, les conditions de l'étape de mise en contact et de l'étape de calcination pouvant être différentes d'une séquence à l'autre. La deuxième étape de mise en contact ou tout autre étape de mise en contact ultérieure peut être une étape de mise en contact du support uniquement avec un précurseur de nickel ou un précurseur de promoteur.

Le solide calciné obtenu est ensuite avantageusement transformé en poudre, notamment par broyage et éventuellement tamisage. De préférence, les grains formant la poudre ont une taille moyenne comprise entre 1 $\mu$m et 1 mm, par exemple entre 100 $\mu$m et 1 mm, notamment entre 200$\mu$m et 800$\mu$m, préférentiellement environ 500 $\mu$m. Le système catalytique peut également se trouver sous forme de billes ayant une taille moyenne comprise entre 1 mm et 5 cm. La détermination de la taille moyenne des grains ou des billes peut être effectuée par granulométrie laser.

L'étape de mise en contact du support avec un précurseur de nickel et un précurseur du promoteur peut être réalisée par exemple par imprégnation, par co-précipitation ou par réaction sol-gel.

Le précurseur de nickel peut être tout composé chimique, ou mélange de composés chimiques, contenant du nickel et plus particulièrement peut être un sel de nickel, un oxyde de nickel ou un mélange de ceux-ci, de préférence un sel de nickel ou un mélange de sels de nickel. Le sel de nickel peut être par exemple choisi parmi le chlorure, le nitrate, le sulfate, le carbonate, l'acétate, l'acétylacétonate, le tartrate, et le citrate de nickel et leurs mélanges, de préférence est le nitrate de nickel, notamment hexahydraté. L'oxyde de nickel peut être un oxyde de nickel (II) ou (III), respectivement notés NiO et $Ni_2O_3$.

Le précurseur du promoteur peut être tout composé chimique, ou mélange de composés chimiques, contenant le métal utilisé comme promoteur et plus particulièrement peut être un sel dudit métal, un oxyde dudit métal ou un mélange de ceux-ci, de préférence un sel dudit métal ou un mélange de sels dudit métal. Le sel dudit métal peut être par exemple choisi parmi le chlorure, le nitrate, le sulfate, le carbonate, l'acétate, l'acétylacétonate, le tartrate, et le citrate dudit métal et leurs mélanges, de préférence le nitrate dudit métal. Dans le cas où le promoteur est un mélange de plusieurs métaux, le précurseur peut être un mélange de différents types de sels et/ou d'oxydes de ces métaux.

Dans le cadre de la présente invention, le terme « sel » désigne un sel non hydraté ou un sel hydraté, voire multi-hydraté.

Selon un premier mode de réalisation, l'étape de mise en contact du support avec un précurseur de nickel et un précurseur du promoteur est réalisée par imprégnation et comprend les étapes suivantes :

> a) préparation d'une solution aqueuse comprenant un précurseur de nickel et un précurseur du promoteur,
> b) ajout du support à la solution aqueuse résultant de l'étape a) pour donner une suspension,
> c) mélange de la suspension résultant de l'étape b),
> d) récupération du solide comprenant le support, le nickel et le promoteur obtenu à l'étape b).

Cette méthode est également dite d'imprégnation par voie humide. Elle consiste à imprégner le support avec le précurseur de nickel et le précurseur du promoteur.

Au cours de l'étape a), une masse appropriée du précurseur de nickel et une masse appropriée du précurseur du promoteur sont ajoutées à un volume approprié d'eau. L'addition de ces précurseurs se fait notamment à température ambiante, c'est-à-dire à une température de 15 à 40°C, avantageusement de 20 à 30°C, notamment de 20 à 25°C. Par « masse appropriée » et « volume approprié », on entend les quantités de précurseurs et d'eau adéquates pour obtenir les teneurs massiques désirées en nickel et en promoteur dans le système catalytique final. Lors de l'étape b), une masse appropriée de support est ajoutée à la solution résultante de l'étape a). Par « masse appropriée », on entend une quantité de support adéquate pour obtenir les teneurs massiques désirées en nickel et en promoteur dans le système catalytique final. Lors de l'étape c), la suspension résultant de l'étape b) est mélangée, notamment par agitation, notamment à température ambiante. Cette étape de mélange pourra être réalisée avantageusement pendant une durée de 30 min ou plus, notamment de 30 min à plusieurs heures, par exemple de 30 min à 3h.

L'étape d) pourra être réalisée par élimination de l'excès d'eau, notamment par évaporation de l'eau ou filtration, puis séchage du solide résultant. L'étape de séchage peut être réalisée par exemple à une température supérieure ou égale à 70°C, par exemple entre 70°C et 150°C, typiquement à une température d'environ 100°C. Ce séchage peut être réalisé pendant 7h ou plus, typiquement pendant une durée allant de 7h à 48h.

Selon un deuxième mode de réalisation, l'étape de mise en contact du support avec un précurseur de nickel et un précurseur du promoteur est réalisée par co-précipitation et comprend les étapes suivantes :

> a') préparation d'une solution aqueuse comprenant un précurseur de nickel et un précurseur du promoteur,
> b') ajout du support à la solution résultant de l'étape a') pour donner une suspension,
> c') ajout d'une base à la suspension résultant de l'étape b') jusqu'à ce que le pH de la suspension soit compris entre 8 et 12, préférentiellement jusqu'à ce que le pH soit d'environ 10,

d') mélange de la suspension résultant de l'étape c'),

e') récupération du solide comprenant le support, le nickel et le promoteur obtenu à l'étape d').

Cette méthode permet de faire précipiter de l'hydroxyde de nickel et de l'hydroxyde du métal utilisé comme promoteur à la surface du support.

Les étapes a') et b') sont réalisées dans les mêmes conditions que les étapes a) et b) respectivement mentionnées ci-dessus.

Lors de l'étape c'), une base est ajoutée à la suspension résultant de l'étape b'). Cette base est avantageusement un sel d'hydroxyde tel que l'hydroxyde de sodium ou de potassium ou le carbonate de sodium ou de potassium notamment l'hydroxyde de sodium. Elle peut être utilisée sous forme d'une solution, notamment aqueuse.

La base est ajoutée jusqu'à ce que le pH de la suspension soit de 10 ou plus, notamment d'environ 10. Cet ajout est progressif, notamment au goutte à goutte. La suspension étant constituée d'un solide en suspension dans une solution liquide, par « pH de la suspension », on entend le pH de la solution liquide de la suspension.

L'étape c') est avantageusement réalisée à une température comprise entre 60°C et 100°C, notamment entre 70°C et 90°C, de préférence égale à environ 80°C.

L'étape d') vise à faire précipiter l'hydroxyde de nickel et l'hydroxyde du métal utilisé comme promoteur à la surface du support. Le mélange, notamment par agitation, est avantageusement réalisé à une température comprise entre 60°C et 100°C, notamment entre 70°C et 90°C, de préférence égale à environ 80°C. Cette étape de mélange peut être réalisée pendant une durée de 2h ou plus, de préférence comprise entre 2h et 5h, typiquement pendant environ 3h.

L'étape e') peut être réalisée comme l'étape d) mentionnée ci-dessus.

Selon un troisième mode de réalisation, l'étape de mise en contact du support avec un précurseur de nickel et un précurseur du promoteur est réalisée par réaction sol-gel et comprend les étapes suivantes :

a") préparation d'une solution comprenant un précurseur de nickel, un précurseur du promoteur et un précurseur de support dans l'acide propionique,

b") chauffage à reflux de la solution de l'étape a"),

c") élimination de l'acide propionique en excès et récupération du système catalytique.

Dans l'étape a"), le précurseur de support correspond à un précurseur de cérium, un précurseur de zirconium ou un mélange de précurseur de cérium et de précurseur de zirconium selon que le support est du cérium, du zirconium ou un mélange de cérium et de zirconium. Dans le cas où le support est un mélange, notamment un oxyde mixte, de cérium et de zirconium, une solution de précurseur de cérium et une solution de précurseur de zirconium sont avantageusement préparées séparément puis mélangées dans un ratio permettant d'obtenir le ratio molaire Ce/Zr désirée dans le mélange, notamment l'oxyde mixte, de cérium et de zirconium final.

Le précurseur de cérium ou de zirconium peut être plus particulièrement un sel de cérium ou de zirconium ou un mélange de sels de cérium ou de zirconium. Le sel de cérium ou de zirconium peut être par exemple choisi parmi le chlorure, le nitrate, le sulfate, le carbonate, l'acétate, l'acétylacétonate, le tartrate, et le citrate de cérium ou de zirconium et leurs mélanges. De préférence, le précurseur de cérium est l'acétate de cérium, notamment sesquihydraté, et le précurseur de zirconium est l'acétylacétonate de zirconium.

Afin de préparer la quantité désirée du catalyseur, le précurseur du support, ainsi que les précurseurs de nickel (e.g. l'acétate tétrahydraté de nickel (II)) et du promoteur sont avantageusement dissous séparément dans de l'acide propionique, notamment avec chauffage (e.g. à 80-120°C, notamment environ 100°C, avantageusement pendant 30 min ou plus, par ex. pendant environ 1h).

L'étape b") de chauffage à reflux est réalisée pendant un temps suffisant pour obtenir des propionates. Par exemple, la solution est chauffée pendant un temps compris entre 1h et 3h. L'étape c") peut être effectuée par évaporation de l'acide propionique. Si le produit résultant de l'étape c") est un gel, il peut être solidifié avant d'effectuer une éventuelle étape de calcination, notamment à l'aide d'azote liquide, de sorte à ce que le produit résultant de l'étape c") soit un solide.

Le support utilisé dans le procédé de préparation du système catalytique peut être un support commercialement disponible.

Selon un mode de réalisation particulier, lorsque l'étape de mise en contact du support avec un précurseur de nickel et un précurseur du promoteur est réalisée par imprégnation ou par co-précipitation, le procédé de préparation du système catalytique comprend la préparation préalable de préparation du support selon les étapes suivantes :

i) préparation d'une solution de précurseur de support dans l'acide propionique,

ii) chauffage à reflux de la solution de l'étape i),

iii) élimination de l'acide propionique en excès, et

iv) calcination du solide résultant de l'étape iii) pour donner le support.

Dans l'étape i), le précurseur de support correspond à un précurseur de cérium, un précurseur de zirconium ou un mélange de précurseur de cérium et de précurseur de zirconium tels que définis ci-dessus pour l'étape a") du procédé de préparation du système catalytique par réaction sol-gel. Dans le cas où le support est un mélange, notamment un oxyde mixte, de cérium et de zirconium, une solution de précurseur de cérium et une solution de précurseur de zirconium sont avantageusement préparées séparément puis mélangées dans un ratio permettant d'obtenir le ratio molaire Ce/Zr désirée dans le mélange, notamment l'oxyde mixte, de cérium et de zirconium final.

L'étape ii) de chauffage à reflux est réalisée pendant un temps suffisant pour obtenir des propionates. Par exemple, la solution est chauffée pendant un temps compris entre 1h et 3h. L'étape iii) peut être effectuée par évaporation de l'acide propionique. Si le produit résultant de l'étape iii) est un gel, il peut être solidifié avant d'effectuer l'étape iv), notamment à l'aide d'azote liquide, de sorte à ce que le produit résultant de l'étape iii) soit un solide.

Le produit résultant de l'étape iii) est alors calciné, par exemple à une température comprise entre 300°C et 600°C°C. Cette étape de calcination peut être réalisée pendant 3h ou plus, par exemple pendant une durée allant de 3h à 6h. Cette étape entraîne la décomposition thermique des propionates afin d'obtenir des oxydes.

*Le procédé de conversion du $CO_2$ et/ou du CO*

La présente invention concerne également un procédé de conversion d'un gaz comprenant du $CO_2$ et / ou du CO en présence d'un système catalytique tel que défini ci-dessus et d'un plasma froid, avantageusement un plasma généré par décharge à barrière diélectrique encore appelé plasma de décharge à barrière diélectrique ou plasma DBD.

Le procédé de conversion d'un gaz comprenant du $CO_2$ et / ou du CO selon l'invention implique la conversion du $CO_2$ et/ou du CO contenus dans ce gaz, notamment en présence de dihydrogène, ce qui implique la réduction et notamment l'hydrogénation du $CO_2$ et/ou du CO. Ce procédé permet ainsi notamment de générer :

- un ou plusieurs hydrocarbures (e.g. méthane), un ou plusieurs alcools (e.g. méthanol, éthanol), du monoxyde de carbone, de l'acide formique et/ou leurs mélanges à partir de la conversion du $CO_2$, et/ou
- un ou plusieurs hydrocarbures (e.g. méthane), un ou plusieurs alcools (e.g. méthanol, éthanol), de l'acide formique et/ou leurs mélanges à partir de la conversion du CO.

Le procédé de conversion d'un gaz comprenant du $CO_2$ et / ou du CO selon l'invention correspond notamment à une hydrogénation du $CO_2$ et / ou du CO respectivement en un ou plusieurs hydrocarbures (e.g. méthane), un ou plusieurs alcools (e.g. méthanol, éthanol), de l'acide formique et/ou un mélange de ceux-ci.

Lorsqu'un hydrocarbure est généré, il s'agit préférablement de méthane, éthane, propane, butane, pentane, hexane ou leurs mélanges, plus préférablement de méthane.

Lorsqu'un alcool est généré, il s'agit préférablement du méthanol, de l'éthanol, du propanol, du butanol ou de leurs mélanges, plus préférablement du méthanol ou de l'éthanol.

De manière préférée, la conversion, notamment l'hydrogénation, du $CO_2$ et/ou du CO selon le procédé de l'invention génère du méthane ou du méthanol, plus préférablement du méthane. Le produit obtenu dépend notamment du ratio molaire entre le $CO_2$ et/ou le CO et le dihydrogène utilisé pour effectuer la réaction de conversion, ratio que l'homme du métier sait parfaitement déterminer en fonction du produit désiré. Par exemple, pour obtenir du méthane à partir de $CO_2$, le ratio $CO_2/H_2$ est de 1 : 4. Pour obtenir du méthanol à partir de $CO_2$, le ratio $CO_2/H_2$ est de 1 : 3. Pour obtenir du CO à partir de $CO_2$, le ratio $CO_2/H_2$ est de 1 : 1.

Le procédé de conversion d'un gaz comprenant du $CO_2$ et/ou du CO selon l'invention comprend le placement du système catalytique selon l'invention dans un dispositif adapté à la génération d'un plasma froid, notamment un plasma DBD. Un tel dispositif comprend avantageusement un réacteur dans lequel sera placé le système catalytique ainsi qu'un système électrique permettant la génération d'un plasma froid, notamment un plasma DBD. Il peut comprendre également un système permettant d'analyser les produits générés par la réaction de conversion du $CO_2$ et/ou du CO.

Les parois du réacteur pourront être dans un matériau métallique ou, dans le cas où un plasma DBD est utilisé, dans un matériau diélectrique tel que le quartz ou une céramique. Le réacteur pourra être avantageusement sous forme cylindrique. Il comprendra notamment au moins une entrée permettant son alimentation en gaz comprenant du $CO_2$ et/ou du CO, et $H_2$ sous forme de gaz et une sortie pour évacuer les produits formés, avantageusement sous forme de gaz. L'entrée du réacteur sera avantageusement connectée à un premier contenant destiné à contenir le gaz comprenant du $CO_2$ et / ou du CO et à un deuxième contenant destiné à contenir le $H_2$. Un système de mélange des gaz (gaz comprenant du $CO_2$ et/ou du CO et $H_2$), notamment dans un ratio $CO_2$ et/ou CO / $H_2$ donné, pourra être présent entre les contenants destinés à recevoir respectivement le gaz comprenant du $CO_2$ et/ou du CO et le $H_2$ et l'entrée du réacteur. La sortie du réacteur sera avantageusement connectée à un contenant destiné à contenir les produits générés par la réaction de conversion. Un système de refroidissement pourra être présent entre le contenant destiné à recevoir les produits générés par la réaction de conversion et la sortie du réacteur de sorte à condenser et éliminer l'eau qui pourrait être formée pendant la réaction de conversion.

Le système électrique permettant la génération de plasma DBD comprend avantageusement deux électrodes, par exemple l'une placée dans le réacteur et l'autre placée à l'extérieur du réacteur, par ex. autour d'une partie du cylindre lorsque le réacteur est cylindrique, entre lesquelles une tension peut être appliquée de sorte à générer un plasma DBD. Le système catalytique sera donc placé à l'intérieur du réacteur, entre ces deux électrodes. Il constitue le lit catalytique. Le système catalytique selon l'invention est activé par la création au niveau du système catalytique (plus particulièrement entre les grains dudit système catalytique) ou à proximité (en amont ou en aval du système catalytique, dans le flux de gaz) d'un fort champ électrique, typiquement entre $10^5$ et $10^{10}$ V /m, qui pourra varier dans le temps. Ce fort champ électrique permet l'ionisation d'une partie du gaz et l'excitation d'atomes et de molécules présents dans la phase gazeuse, typiquement les molécules de CO et/ou de $CO_2$ et de $H_2$, par les électrons ainsi arrachés et accélérés. Tandis que la température des électrons est de plusieurs milliers ou de plusieurs dizaines de milliers de kelvins, la majorité du gaz reste, elle, à température ambiante ou à quelques centaines de kelvins. C'est pourquoi on parle de plasma « froid » ou « non thermique » (qui n'est pas à l'équilibre thermodynamique).

En fonction de la vitesse de variation de ce fort champ électrique, le plasma pourra être qualifié de radio-fréquence, de micro-ondes (selon les fréquences correspondantes à ces gammes électromagnétiques), ou plus avantageusement de basse fréquence (50 Hz < f <1 MHz) ou d'impulsionnel (impulsions de durées comprises entre 1 ns et 1 ms, avec des temps de montée rapides de 0.1 ns à 100 $\mu$s).

Dans ces deux derniers cas, deux électrodes seront notamment placées de part et d'autre du catalyseur et un générateur de haute tension qui créera une différence de potentiel entre ces électrodes afin de créer le champ électrique désiré, qu'il soit alternatif, basse fréquence ou pulsé. Avantageusement, si le réacteur est de forme cylindrique, une électrode peut être placée à l'intérieur du réacteur et l'autre à l'extérieur du réacteur. Le système catalytique sera alors placé à l'intérieur du réacteur, entre ces deux électrodes. Il constitue le lit catalytique.

Dans le cas spécifique d'un plasma DBD, au moins une couche de matériau diélectrique, préférentiellement deux, sont insérées entre les deux électrodes. Ces matériaux diélectriques peuvent, ou non, être également les parois du réacteur catalytique.

En plus de la création d'un état de plasma froid, le fort champ électrique créé est responsable de la polarisation négative ou positive des sites catalytiques. Cette polarisation induit des réactions d'adsorption et de désorption même à basse température (par exemple à des températures inférieures à 300°C, voire inférieure à 200°C). Sans polarisation (procédé conventionnel), la température de travail est plus élevée, généralement de 300°C à 420°C. Dans une variante de l'invention, le système catalytique de l'invention est activé par un plasma DBD en fournissant une puissance électrique de 0.001 W/g à 3 W/g de système catalytique (à savoir le système catalytique présent dans le réacteur, c'est-à-dire dans le lit catalytique).

Avant l'étape de conversion, notamment d'hydrogénation, le système catalytique selon l'invention, dans le cas où ses composants sont sous forme oxydés, est avantageusement réduit *in situ* sous plasma froid, préférablement un plasma DBD, sous $H_2$ comme gaz de décharge.

La réaction de conversion est avantageusement réalisée à une pression supérieure ou égale à la pression atmosphérique ($10^5$ Pa), par exemple à une pression allant de la pression atmosphérique à $3.10^5$ Pa. La réaction de conversion peut être réalisée dans des conditions pseudo-adiabatiques, c'est-à-dire sans isolation thermique et sans chauffage externe, ou dans des conditions isothermes, c'est-à-dire avec isolation thermique.

Dans le cas d'un plasma DBD, ce dernier est avantageusement généré entre les électrodes du système électrique permettant de générer le plasma DBD par application d'une tension entre les deux électrodes, comprise entre 1 et 25kV, préférablement entre 8 et 16 kV, notamment avec une fréquence de comprise entre 1 kHz à 100 kHz.

La température du lit catalytique dépend de la tension appliquée. Typiquement cette température est inférieure à 300°C, en particulier elle est comprise entre 150°C et 270°C. La vitesse spatiale horaire du gaz (GHSV) est notamment comprise entre 1000 $h^{-1}$ et 100000 $h^{-1}$, préférablement entre 30000 $h^{-1}$ et 50000 $h^{-1}$.

Lors de la réaction de conversion, notamment d'hydrogénation, du gaz comprenant du $CO_2$ et/ou du CO à l'aide d'un procédé hybride catalyse/plasma froid en utilisant le système catalytique selon l'invention, le taux de conversion du $CO_2$ et/ou du CO observé est typiquement supérieur à 70% avec une puissance consommée inférieure à 5W, et même supérieure à 80% avec une puissance consommée inférieure à 8W. La sélectivité envers le produit désiré (par exemple le méthane si la réaction de conversion est une réaction de méthanation) est typiquement supérieure à 95%, et même supérieure à 99%.

**EXEMPLES**

**A) Matériels et méthodes**

**I. Préparation des systèmes catalytiques**

*Supports :*

Plusieurs supports ont été utilisés :

1) Un oxyde mixte de Cérium-Zirconium commercial ($Ce_xZr_{1-x}O_2$, x = 0,58, Solvay) ;
2) Un oxyde de cérium $CeO_2$ commercial (Sigma-Aldrich) ;
3) Un oxyde de zirconium $ZrO_2$ commercial (Daiichi Kigenso kagaku Kogyo Co) ;
4) Un oxyde mixte de Cérium-Zirconium de composition molaire $Ce_{0,58}Zr_{0,42}O_2$, synthétisé par les inventeurs selon la méthode décrite ci-après. Ce support à la même composition que le support 1).

*Synthèse du support 4) de type oxyde mixte cérium-zirconium de composition molaire $Ce_{0,58}Zr_{0,42}O_2$ (CZ) :*

Pour la synthèse du support de type CZ, les sels organométalliques de départ qui ont été utilisés sont l'acétate sesquihydraté de cérium (III) et l'acétylacétonate de zirconium (IV). Ces sels sont dissouts séparément dans de l'acide propionique à chaud pendant 1 h, de manière à obtenir des solutions de concentration de 0,12 mol.$L^{-1}$. Il est indispensable de dissoudre les sels de départ appropriés, qui conduiront à l'obtention exclusive des propionates métalliques désirés. Les différentes solutions sont alors mélangées et chauffées sous reflux dans un ballon équipé d'un réfrigérant à boules durant 90 min ($T_{eb}$ acide propionique = 141°C), donnant naissance à des propionates mixtes. Le solvant est ensuite évaporé via une distillation contrôlée sous vide jusqu'à l'obtention par oligomérisation d'un gel mixte, contenant les éléments métalliques dans la stœchiométrie choisie. Ce gel est solidifié grâce à de l'azote liquide et calciné sous air pendant 6 h, à une température de 400 à 600°C, généralement à 500 °C, en utilisant une rampe de température de 2°C/min. Cette étape entraîne la décomposition thermique des propionates afin d'obtenir l'oxyde mixte ayant la composition désirée.

*Synthèse des systèmes catalytiques :*

Des systèmes catalytiques ont été synthétisés par imprégnation par voie humide ou par co-précipitation.

Les systèmes catalytiques ont été préparés à partir des trois supports décrits ci-dessus (oxyde de cérium, oxyde de zirconium ou oxyde mixte cérium et zirconium) avec 15% en poids de Nickel par rapport au poids du support. Les promoteurs suivants ont été investigués, pour certains avec différentes teneurs massiques au sein du système catalytique : le gadolinium (Gd), l'yttrium (Y), le strontium (Sr), le lanthane (La), le praséodyme (Pr), le manganèse (Mn), le cobalt (Co), le magnésium (Mg), le zinc (Zn), le potassium (K), l'indium (In), le fer (Fe), le sodium (Na) et le cuivre (Cu).

Ainsi, différentes natures de support, différentes méthodes de préparation du système catalytique, différents promoteurs et différentes quantités de promoteurs ont été étudiées.

*a) Préparation du système catalytique par imprégnation par voie humide*

Les systèmes catalytiques au nickel dopés par le promoteur (métal M) ont été préparés par la méthode de co-imprégnation par voie humide à partir d'une solution aqueuse de $Ni(NO_3)_2.6H_2O$ (Sigma-Aldrich) et d'un précurseur de promoteur choisi parmi : $Cu(NO_3)_2.5H_2O$, $Co(NO_3)_2.6H_2O$, $Mn(NO_3)_2.4H_2O$, $La(NO_3)_2.6H_2O$, $Y(NO_3)_2.6H_2O$, $Sr(NO_3)_2.4H_2O$, $Gd(NO_3)_2.6H_2O$, $Mg(NO_3)_2.6H_2O$, $Zn(NO_3)_2.6H_2O$, $NaNO_3$, $KNO_3$, et $In(NO_3)_2.6H_2O$ (tous commerciaux, Sigma-Aldrich), selon le promoteur désiré.

La teneur en nickel est de 15 % massique par rapport au poids du support et celle en promoteur M varie entre 2, 4, 7, 10, 15 et 20% massique par rapport au support. Un catalyseur sans promoteur a également été préparé comme matériau de référence.

La masse de sel de nickel appropriée et celle du précurseur de promoteur sont dissoutes dans un volume d'eau de 250 mL, à température ambiante et sous agitation. La masse adéquate de support est ajoutée à la solution aqueuse contenant le mélange des sels métalliques et maintenue sous agitation pendant 2 heures. Par exemple, 3,72g de nitrate de nickel (teneur 15% en poids), 1,5 g de nitrate d'yttrium (teneur 7% en poids) et 5 g de support sont mélangés selon la procédure précédente. Le mélange est alors placé dans un évaporateur rotatif pendant 2h à 60°C, afin d'éliminer l'excès d'eau.

Après imprégnation et évaporation de l'eau, tous les échantillons ont été collectés, séchés dans un four à 100°C pendant 15 h, puis calcinés sous air à 550°C pendant 4 h avec une rampe de température de 10°C/min. Après calcination, les échantillons de système catalytique ont été broyés à la main et tamisés jusqu'à une taille de grain moyenne comprise entre 10 et 50 $\mu$m. Après cette étape, plusieurs catalyseurs de différentes tailles de particules ont ensuite été fabriqués selon la procédure suivante. Tout d'abord, les poudres catalytiques originales ont été pressées dans un comprimé à l'aide d'un moule, puis le comprimé a été broyé en petits morceaux. Finalement, les particules de catalyseur ont été séparées en différentes tailles à l'aide de plusieurs mailles qui varient entre 30 $\mu$m et 5 mm. Les meilleures performances catalytiques et énergétiques ont été obtenues pour une taille de particules d'environ 500 $\mu$m.

La distribution de la taille des grains a été certifiée par granulométrie laser.

*b) Préparation du système catalytique par co-précipitation*

Le système catalytique préparé par co-précipitation comprend un support d'oxyde mixte de cérium et de zirconium commercial sous forme de poudre ($Ce_xZr_{1-x}O_2$, x = 0,58, Solvay) désigné sous le nom de CZ.

Les systèmes catalytiques de type NiCZ-M (M étant le promoteur) ont été préparés avec une teneur massique de Ni de 15% par rapport au support. À titre de comparaison, un catalyseur de type NiCZ (sans promoteur) à 15 % en poids de nickel par rapport au poids du support a également été préparé par la même méthode.

La poudre de CZ a d'abord été mise en suspension dans la solution aqueuse contenant une quantité appropriée de nitrate de nickel et de nitrate du promoteur désiré à température ambiante. Afin de synthétiser la quantité désirée de catalyseur, une solution de NaOH à 2M a été également préparée. Cette dernière solution a été ajoutée goutte à goutte sur le mélange contenant la poudre de CZ avec les nitrates de nickel et de promoteur, à une température de 80°C et sous agitation, jusqu'à ce que la valeur du pH de la solution mélangée atteigne 10. Ensuite, ce mélange a été vieilli pendant 3 h sous agitation vigoureuse à 80°C, pendant laquelle l'hydroxyde de nickel et de promoteur a été exclusivement précipité à la surface du support. Le pH et la température sont contrôlés tout au long de la synthèse avec un testeur de pH et de température étanche. Le solide ainsi obtenu est ensuite filtré et lavé avec de l'eau distillée et séché à l'étuve à 100°C pendant une nuit. Le solide ainsi obtenu est calciné sous air à 550°C pendant 4 h avec une rampe de température de 10°C/min. Après calcination, les échantillons de système catalytique ont été broyés à la main et tamisés jusqu'à une taille moyenne de grain comprise entre 10 et 50 $\mu$m. Après cette étape, plusieurs catalyseurs de différentes tailles de particules ont ensuite été fabriqués selon la procédure détaillée au paragraphe a).

*c) Préparation du système catalytique par imprégnation séquencée*

Le système catalytique est préparé par deux séquences d'imprégnation successives. En d'autres termes, une première imprégnation par voie humide du support avec le nickel et le promoteur désiré est réalisée selon la méthode a) ci-dessus jusqu'à l'étape de calcination. Après la calcination, une nouvelle imprégnation uniquement du promoteur cette fois est réalisée sur le système catalytique issu de la première imprégnation.

## II. Conditions et paramètres expérimentaux de l'hydrogénation du $CO_2$ : exemple de la méthanation du $CO_2$

L'activité et la sélectivité des systèmes catalytiques avec et sans promoteur dans la méthanation du $CO_2$, dans des conditions de plasma DBD, ont été évaluées dans un dispositif expérimental comprenant un réacteur cylindrique tubulaire en quartz, un générateur de plasma DBD, et les dispositifs correspondants pour l'alimentation et l'analyse des gaz. Une représentation schématique du dispositif de plasma DBD est fournie en Figure 1.

Un plasma de décharge à barrière diélectrique (DBD) non thermique a été créé entre deux électrodes : une électrode cylindrique en cuivre placée à l'intérieur d'un tube en alumine (3 mm de diamètre), entourée d'un tube en quartz coaxial (10 mm de diamètre interne, 1 mm d'épaisseur), et un fil d'acier enroulé autour de la surface extérieure du tube en quartz, agissant comme électrode de masse (mise à la terre via un condensateur externe de 2 nF). Dans cette configuration, une décharge était soutenue dans un espace de 2,5 mm, couvrant une longueur d'environ 6,5 mm. Avant chaque expérience de méthanation, le réacteur adiabatique tubulaire est chargé de 300 mg de système catalytique (taille de grain d'environ 30 $\mu$m) formant le lit catalytique. Des deux côtés du lit catalytique, de la laine de verre a été utilisée pour maintenir le système catalytique fixé dans la zone de décharge. Avant l'essai catalytique, les systèmes catalytiques tels que synthétisés ont été réduits *in situ* sous plasma DBD non thermique sous $H_2$ comme gaz de décharge (tension = 15,0 kV, fréquence = 70,3 kHz, débit = 160 ml.min$^{-1}$CNTP) pour une durée de 60 minutes. Par la suite, dans les conditions du plasma DBD, la méthanation du $CO_2$ a été effectuée à pression ambiante (P= 1 atm.), dans des conditions pseudo-adiabatiques, avec un débit de 200 mL/min d'un mélange gazeux contenant du $CO_2$ et de l'$H_2$ ($H_2$ : $CO_2$ = 4 :1). Ces conditions d'écoulement correspondent à une vitesse spatiale horaire du gaz (GHSV) égale à 44 000 h$^{-1}$.

Les expériences de plasma-catalyse ont été réalisées à une fréquence de 70 kHz, et à des tensions comprises entre 13 et 16 kV. La tension appliquée a été mesurée à l'aide d'un oscilloscope pour PC (Picoscope série 5000, technologie Pico), avec une sonde (ELDITEST GE 3830). La méthode de Lissajou (11)-(12) a été utilisée pour la détermination de la puissance du plasma d'entrée. Toutes les expériences ont été réalisées dans des conditions pseudo-adiabatiques (sans isolation thermique) et sans chauffage externe (ni le réacteur, ni l'entrée du gaz n'est chauffée). La température, mesurée avec un thermocouple de type K situé sur l'électrode de terre près du lit catalytique, dépend fortement de la tension appliquée. L'eau liquide formée par la réaction de méthanation est éliminée dans un condensateur. Le débit de gaz sortant de ce condenseur et entrant dans l'appareil d'analyse de gaz est régulièrement mesuré à l'aide d'un débitmètre à bulles. Les concentrations de $CO_2$, $H_2$, $CH_4$ et CO dans le gaz sortant du condenseur ont été mesurées à l'aide d'un chromatographe en phase gazeuse (IGC A20 ML, Delsi Intersmat) équipé d'une colonne Carboxen et d'un détecteur de conductivité thermique (TCD).

Les équations suivantes ont été utilisées pour le calcul de la conversion du $CO_2$, de la sélectivité du $CH_4$ et de la sélectivité du CO, le tout en pourcentage :

$$X_{CO_2}(\%) = \frac{F_{CO_2}^{in} - F_{CO_2}^{out}}{F_{CO_2}^{in}} \times 100$$

$$S_{CH_4}(\%) = \frac{F_{CH_4}^{out}}{F_{CO_2}^{in} - F_{CO_2}^{out}} \times 100$$

$$S_{CO}(\%) = \frac{F_{CO}^{out}}{F_{CO_2}^{in} - F_{CO_2}^{out}} \times 100$$

où $F^{in}$ et $F^{out}$ désignent respectivement le débit d'entrée et de sortie (mol/s) de l'espèce considérée à l'entrée et à la sortie du réacteur.

## B) Résultats

La performance catalytique et la consommation d'énergie ont été évaluées pour chaque système catalytique synthétisé dans la réaction de méthanation du $CO_2$ utilisant un plasma DBD selon la méthode décrite ci-dessus.

### I. La nature du promoteur

Des catalyseurs à base de support d'oxyde mixte CeZr avec 15% en poids de nickel par rapport au poids du support et différents promoteurs ont été testés. La teneur massique en promoteur dans le système catalytique est de 4% en poids par rapport au poids du support. Les résultats de leur performance catalytique et de la consommation d'énergie selon la nature du promoteur introduit dans le système catalytique sont résumés dans le tableau 1 ci-dessous. Les catalyseurs des entrées 1, 2, 3, 4, 5, 10, 11, 13 et 14 correspondent à la présente invention.

Tableau 1

| Entrée | Promoteur | Catalyseur | Taux de conversion du $CO_2$ (%) | Sélectivité vers le méthane (%) | Performances énergétiques |
|--------|-----------|------------|----------------------------------|----------------------------------|---------------------------|
| 0 | - | NiCeZr | - | ++ | - |
| (témoin) | | (référence) | 48±3 | 93±1 | |
| 1 | Y | NiCeZrY | +++ 76±3 | +++ 97±1 | +++ |
| 2 | Gd | NiCeZrGd | +++ 83±3 | +++ 98 ± 1 | +++ |
| 3 | Co | NiCeZrCo | ++ 74±3 | ++ 94±1 | +++ |
| 4 | Sr | NiCeZrSr | ++ 71 ±3 | ++ 91 ±1 | ++ |
| 5 | Mn | NiCeZrMn | ++ 63 ±3 | ++ 95 ±1 | ++ |
| 6 | Mg | NiCeZrMg | + 55 ±3 | ++ 92 ±1 | - |
| 7 | Zn | NiCeZrZn | 32±3 | - | - |
| 8 | K | NiCeZrK | 19±3 | - | - |

(suite)

| Entrée | Promoteur | Catalyseur | Taux de conversion du $CO_2$ (%) | Sélectivité vers le méthane (%) | Performances énergétiques |
|---|---|---|---|---|---|
| 9 | In | NiCeZrIn | 17±3 | - | - |
| 10 | Pr | NiCeZrPr | ++ 72 ± 3 | +++ 96 ±1 | ++ |
| 11 | Fe | NiCeZrFe | + 53 ±3 | ++ 94 ±1 | + |
| 12 | Na | NiCeZrNa | 18±3 | - | - |
| 13 | La | NiCeZrLa | + 58 ±3 | ++ 94 ±1 | ++ |
| 14 | Cu | NiCeZrCu | + 53 ±3 | ++ 94 ±1 | + |

La performance catalytique est notamment évaluée en fonction du taux de conversion à une puissance de référence de 8 W. Une performance catalytique +++ correspond à un taux de conversion du $CO_2$ supérieur ou égal à 75%. Une performance catalytique ++ correspond à un taux de conversion du $CO_2$ supérieur ou égal à 60%. Une performance catalytique + correspond à un taux de conversion du $CO_2$ supérieur ou égal à 50%. Une performance catalytique - correspond à un taux de conversion inférieur à 50%.

Une sélectivité du méthane +++ correspond à une sélectivité du méthane supérieur ou égal à 95%. Une sélectivité du méthane ++ correspond à une sélectivité du méthane supérieure ou égal à 90%. Une sélectivité du méthane - correspond à une sélectivité du méthane inférieure à 90%.

La performance énergétique est évaluée en fonction de la puissance consommée pour obtenir un taux de conversion d'au moins 60%. Une performance énergétique +++ correspond à une puissance consommée inférieure ou égale à 6 W. Une performance énergétique ++ correspond à une puissance consommée inférieure ou égale à 9 W. Une performance énergétique + correspond à une puissance consommée inférieure ou égale à 12W. Une performance énergétique - correspond à une puissance consommée supérieur à 12 W.

Le taux de conversion obtenu en fonction de la puissance consommée est représenté pour chaque système catalytique selon l'invention en Figure 2 (A). La Figure 2 (B) quant à elle représente la sélectivité envers le méthane en fonction de la puissance consommée pour chaque catalyseur selon l'invention.

L'indium, le zinc, le sodium, le potassium et le fer n'apportent aucune amélioration en termes de performances catalytique et énergétique par rapport au catalyseur témoin sans promoteur. En revanche, on observe que les promoteurs selon l'invention permettent d'améliorer les performances catalytique (le taux de conversion et la sélectivité) et énergétique de la réaction de méthanation, en particulier le gadolinium et l'yttrium.

En utilisant le catalyseur témoin NiCZ sans promoteur, un maximum de conversion de $CO_2$ obtenu est de 73% à 250°C avec une consommation d'énergie de 14,5 W. Avec l'ajout d'un promoteur selon l'invention, ce taux de conversion peut être obtenu à une moindre puissance. Par exemple, l'ajout de gadolinium permet d'obtenir une conversion de $CO_2$ de 73-74% à 3,5-3,7W au lieu de 14,5 W avec le catalyseur NiCZ sans promoteur.

Ainsi, les systèmes catalytiques selon l'invention présentent l'avantage d'un gain de 10-12% du taux de conversion par rapport aux systèmes catalytiques à base de nickel et d'oxyde mixte cérium-zirconium déjà existant, avec une sélectivité pouvant atteindre 100% en méthane, à pression atmosphérique et à une température entre 180 et 230°C, avec une consommation d'énergie quatre fois inférieure.

**II. La teneur massique en promoteur**

Des systèmes catalytiques à base de support d'oxyde mixte CeZr avec 15% en poids de nickel par rapport au poids du support et de l'yttrium et/ou du gadolinium en tant que promoteur à différentes teneurs massiques ont été testés. La teneur massique est exprimée en poids de promoteur par rapport au poids du support.

Les résultats de leur performance catalytique et de la consommation d'énergie selon la teneur massique du promoteur introduit dans le système catalytique sont résumés dans le tableau 2 ci-dessous.

Tableau 2

| Entrée | Promoteur | Catalyseur | Teneur en promoteur (w/w%) | Taux de conversion du $CO_2$ (%) | Sélectivité vers le méthane (%) | Performances énergétiques |
|---|---|---|---|---|---|---|
| 0 (témoin) | - | NiCeZr (référence) | 0 | - 48±3 | ++ 93±1 | - |

(suite)

| Entrée | Promoteur | Catalyseur | Teneur en promoteur (w/w%) | Taux de conversion du $CO_2$ (%) | Sélectivité vers le méthane (%) | Performances énergétiques |
|---|---|---|---|---|---|---|
| 1 (comp) | Y | NiCeZrY-4 | 4 | +++ 76 ±3 | +++ 97±1 | ++ |
| 2 (comp) | Y | NiCeZrY-7 | 7 | +++ 78±3 | +++ 98±1 | +++ |
| 3 (comp) | Y | NiCeZrY-10 | 10 | + 56±3 | ++ 95±1 | ++ |
| 4 (inv) | Gd | NiCeZrGd-2 | 2 | +++ 76 ±3 | +++ 97±1 | ++ |
| 5 (inv) | Gd | NiCeZrGd-4 | 4 | +++ 83 ±3 | +++ 98±1 | +++ |
| 6 (inv) | Gd | NiCeZGd-7 | 7 | ++ 73 ±3 | +++ 95±1 | ++ |
| 7 (inv) | Gd | NiCeZrGd-10 | 10 | ++ 70 ±3 | ++ 94±1 | ++ |
| 8 (inv) | Gd | NiCeZrGd-15 | 15 | + 59 ±3 | + 92±1 | + |
| 9 (inv) | Gd et Y | NiCeZrGd-4-Y-7 | Gd- 4 et Y-7 | ++ 72±3 | ++ 95±1 | ++ |
| Comp : comparatif - Inv : selon l'invention | | | | | | |

La performance catalytique est notamment évaluée en fonction du taux de conversion à une puissance de référence de 8 W. Une performance catalytique +++ correspond à un taux de conversion du $CO_2$ supérieur ou égal à 75%. Une performance catalytique ++ correspond à un taux de conversion du $CO_2$ supérieur ou égal à 60%. Une performance catalytique + correspond à un taux de conversion du $CO_2$ supérieur ou égal à 50%. Une performance catalytique - correspond à un taux de conversion inférieur à 50%.

Une sélectivité du méthane +++ correspond à une sélectivité du méthane supérieur ou égal à 95%. Une sélectivité du méthane ++ correspond à une sélectivité du méthane supérieure ou égal à 90%. Une sélectivité du méthane - correspond à une sélectivité du méthane inférieure à 90%.

La performance énergétique est évaluée en fonction de la puissance consommée pour obtenir un taux de conversion d'au moins 60%. Une performance énergétique +++ correspond à une puissance consommée inférieure ou égale à 6 W. Une performance énergétique ++ correspond à une puissance consommée inférieure ou égale à 9 W. Une performance énergétique + correspond à une puissance consommée inférieure ou égale à 12W. Une performance énergétique - correspond à une puissance consommée supérieur à 12 W.

Le taux de conversion obtenu en fonction de la puissance consommée est représenté pour chaque catalyseur à base d'yttrium du tableau 2 en Figure 3 (A). La Figure 3 (B) quant à elle représente la sélectivité envers le méthane par rapport au CO en fonction de la puissance consommée pour chaque catalyseur à base d'yttrium.

A toutes les teneurs massiques étudiées, et particulièrement à des teneurs massiques comprises entre 0,1% et 10°% en poids par rapport au poids du support, l'introduction du promoteur améliore les performances catalytiques et énergétiques de la réaction par rapport au catalyseur de référence sans promoteur. Pour les catalyseurs à base de gadolinium, les meilleurs résultats sont obtenus avec une teneur massique de 4%. Pour les catalyseurs à base d'yttrium, les meilleurs résultats sont obtenus avec une teneur massique de 7%.

### III. La nature du support

Des catalyseurs hors invention à base de support d'oxyde mixte CeZr, de support d'oxyde de cérium $CeO_2$ ou de support d'oxyde de zirconium $ZrO_2$ avec 15% en poids de nickel et 7% en poids d'yttrium en tant que promoteur poids par rapport au poids du support ont été testés. Les systèmes catalytiques témoins (entrées 0, 2 et 4) ne comprennent pas de promoteur. Les résultats de leur performance catalytique et de la consommation d'énergie selon la nature du support dans le système catalytique sont résumés dans le tableau 3 ci-dessous.

Tableau 3

| Entrée | Support | Catalyseur | Taux de conversion du $CO_2$ (%) | Sélectivité vers le méthane (%) | Performances énergétiques |
|---|---|---|---|---|---|
| 1 (témoin) | CeZr | NiCeZr (référence) | + 62±3 | +++ 97±1 | - |
| 2 | CeZr | NiCeZrY-7 | +++ 79±3 | +++ 95±1 | +++ |

(suite)

| Entrée | Support | Catalyseur | Taux de conversion du $CO_2$ (%) | Sélectivité vers le méthane (%) | Performances énergétiques |
|---|---|---|---|---|---|
| 3 (témoin) | $CeO_2$ | $NiCeO_2$ (référence) | ++ 67±3 | ++ 94±1 | + |
| 4 | $CeO_2$ | $NiCeO_2$Y-7 | ++ 72±3 | +++ 95±1 | ++ |
| 5 (témoin) | $ZrO_2$ | $NiZrO_2$ (référence) | + 59±3 | ++ 92±1 | ++ |
| 6 | $ZrO_2$ | $NiZrO_2$Y-7 | ++ 68±3 | ++ 94±1 | ++ |

La performance catalytique est notamment évaluée en fonction du taux de conversion à une puissance de référence de 10 W. Une performance catalytique +++ correspond à un taux de conversion du $CO_2$ supérieur ou égal à 75%. Une performance catalytique ++ correspond à un taux de conversion du $CO_2$ supérieur ou égal à 60%. Une performance catalytique + correspond à un taux de conversion du $CO_2$ supérieur ou égal à 50%. Une performance catalytique - correspond à un taux de conversion inférieur à 50%.

Une sélectivité du méthane +++ correspond à une sélectivité du méthane supérieur ou égal à 95%. Une sélectivité du méthane ++ correspond à une sélectivité du méthane supérieure ou égal à 90%. Une sélectivité du méthane - correspond à une sélectivité du méthane inférieure à 90%.

La performance énergétique est évaluée en fonction de la puissance consommée pour obtenir un taux de conversion d'au moins 60%. Une performance énergétique +++ correspond à une puissance consommée inférieure ou égale à 6 W. Une performance énergétique ++ correspond à une puissance consommée inférieure ou égale à 9 W. Une performance énergétique + correspond à une puissance consommée inférieure ou égale à 12W. Une performance énergétique - correspond à une puissance consommée supérieur à 12 W.

Avec les trois supports différents, l'introduction d'un promoteur permet d'améliorer les performances catalytiques et énergétiques de la réaction. Le support à base d'oxyde mixte cérium-zirconium permet d'obtenir les meilleurs résultats.

### IV. La méthode de préparation utilisée

Des catalyseurs à base de support d'oxyde mixte CeZr avec 15% en poids de nickel et 7% en poids d'yttrium (hors invention) ou 4% en poids de gadolinium (selon l'invention) en tant que promoteur poids par rapport au poids du support obtenus via différentes méthodes de préparation ont été testés.

Les résultats de leur performance catalytique et de la consommation d'énergie selon la nature du support dans le système catalytique sont résumés dans le tableau 4 ci-dessous.

Tableau 4

| Entrée | Catalyseur | Méthode de préparation | Taux de conversion du $CO_2$ (%) | Sélectivité vers le méthane (%) | Performances énergétiques |
|---|---|---|---|---|---|
| 0 (témoin) | NiCeZr (reference) | Imprégnation par voie humide | - 48±3 | ++ 93±1 | |
| 1 (témoin) | NiCeZr (reference) | Co-précipitation | - 50±3 | ++ 93±1 | - |
| 2 (comp) | NiCeZrY-7 | Imprégnation par voie humide | +++ 78±3 | +++ 98±1 | +++ |
| 3 (comp) | NiCeZrY-7 | Co-précipitation | ++ 73±3 | ++ 95±1 | ++ |
| 4 (comp) | NiCeZrY-7 | Imprégnation séquencée | ++ 69±3 | ++ 95±1 | ++ |
| 5 (comp) | NiCeZrGd-4 | Imprégnation par voie humide | +++ 83 ±3 | +++ 98±1 | +++ |
| 6 (inv) | NiCeZrGd-4 | Co-précipitation | +++ 76±3 | +++ 96±1 | +++ |
| 7 (inv) | NiCeZrGd-4 | Imprégnation séquencée | ++ 72±3 | +++ 95±1 | +++ |
| Comp : comparatif - Inv : selon l'invention | | | | | |

La performance catalytique est notamment évaluée en fonction du taux de conversion à une puissance de référence de 8 W. Une performance catalytique +++ correspond à un taux de conversion du $CO_2$ supérieur ou égal à 75%.

Une performance catalytique ++ correspond à un taux de conversion du $CO_2$ supérieur ou égal à 60%. Une performance catalytique + correspond à un taux de conversion du $CO_2$ supérieur ou égal à 50%. Une performance catalytique - correspond à un taux de conversion inférieur à 50%.

Une sélectivité du méthane +++ correspond à une sélectivité du méthane supérieur ou égal à 95%. Une sélectivité du méthane ++ correspond à une sélectivité du méthane supérieure ou égal à 90%. Une sélectivité du méthane - correspond à une sélectivité du méthane inférieure à 90%.

La performance énergétique est évaluée en fonction de la puissance consommée pour obtenir un taux de conversion d'au moins 60%. Une performance énergétique +++ correspond à une puissance consommée inférieure ou égale à 6 W. Une performance énergétique ++ correspond à une puissance consommée inférieure ou égale à 9 W. Une performance énergétique + correspond à une puissance consommée inférieure ou égale à 12W. Une performance énergétique - correspond à une puissance consommée supérieur à 12 W.

Quelle que soit la méthode de préparation utilisée, l'introduction d'un promoteur permet d'améliorer les performances catalytiques et énergétiques par rapport au catalyseur sans promoteur. La méthode par imprégnation par voie humide donne les meilleurs résultats.

Références :

(1) F. Ocampo, B. Louis, et A.-C. Roger, « Methanation of carbon dioxide over nickelbased Ce0.72Zr0.28O2 mixed oxide catalysts prepared by sol-gel method », Applied Catalysis A: General, vol. 369, no 1-2, p. 90-96, nov. 2009, doi: 10.1016/j.apcata.2009.09.005.

(2) M. Nizio, A. Albarazi, S. Cavadias, J. Amouroux, M. E. Galvez, et P. Da Costa, « Hybrid plasma-catalytic methanation of CO2 at low temperature over ceria zirconia supported Ni catalysts », International Journal of Hydrogen Energy, vol. 41, no 27, p. 11584-11592, juill. 2016, doi: 10.1016/j.ijhydene.2016.02.020.

(3) J. Amouroux et S. Cavadias, « Electrocatalytic reduction of carbon dioxide under plasma DBD process », J. Phys. D: Appl. Phys., vol. 50, no 46, p. 465501, nov. 2017, doi: 10.1088/1361-6463/aa8b56.

(4) M. Mikhail et al., « Plasma-catalytic hybrid process for CO2 methanation: optimization of operation parameters », Reac Kinet Mech Cat, vol. 126, no 2, p. 629-643, avr. 2019, doi: 10.1007/s11144-018-1508-8.

(5) M. Mikhail et al., « Electrocatalytic behaviour of CeZrOx-supported Ni catalysts in plasma assisted CO2 methanation », Catal. Sci. Technol., vol. 10, no 14, p. 4532-4543, 2020, doi: 10.1039/D0CY00312C.

(6) M. Nizio et al., « Low temperature hybrid plasma-catalytic methanation over Ni-Ce-Zr hydrotalcite-derived catalysts », Catalysis Communications, vol. 83, p. 14-17, août 2016, doi: 10.1016/j.catcom.2016.04.023.

(7) E. Jwa, S. B. Lee, H. W. Lee, et Y. S. Mok, « Plasma-assisted catalytic methanation of CO and CO2 over Ni-zeolite catalysts », Fuel Processing Technology, vol. 108, p. 89-93, avr. 2013, doi: 10.1016/j.fuproc.2012.03.008.

(8) M. Biset-Peirô, J. Guilera, T. Zhang, J. Arbiol, et T. Andreu, « On the role of ceria in Ni-Al2O3 catalyst for CO2 plasma methanation », Applied Catalysis A: General, vol. 575, p. 223-229, avr. 2019, doi: 10.1016/j.apcata.2019.02.028.

(9) M. C. Bacariza et al., « DBD plasma-assisted CO2 methanation using zeolite-based catalysts: Structure composition-reactivity approach and effect of Ce as promoter », Journal of CO2 Utilization, vol. 26, p. 202-211, juill. 2018, doi: 10.1016/j.jcou.2018.05.013.

(10) Amouroux J et al., 2016, European Patent 3050865A1.

(11) T. C. Manley, « The Electric Characteristics of the Ozonator Discharge », Trans. Electrochem. Soc., vol. 84, no 1, p. 83, 1943, doi: 10.1149/1.3071556.

(12) R. Valdivia-Barrientos, J. Pacheco-Sotelo, M. Pacheco-Pacheco, J. S. Benítez-Read, et R. Lôpez-Callejas, « Analysis and electrical modelling of a cylindrical DBD configuration at different operating frequencies », Plasma Sources Sci. Technol., vol. 15, no 2, p. 237-245, mai 2006, doi: 10.1088/0963-0252/15/2/008.

**Revendications**

1. Procédé de conversion d'un gaz comprenant du $CO_2$ et/ ou du CO en présence d'un système catalytique et d'un plasma froid, de préférence un plasma généré par décharge à barrière diélectrique (DBD), ledit système catalytique comprenant :

   - un support comprenant du cérium et/ou du zirconium,
   - du nickel, et
   - un promoteur choisi parmi le gadolinium et les mélanges gadolinium et yttrium.

**2.** Procédé selon la revendication 1, **caractérisé en ce que** le promoteur est le gadolinium.

**3.** Procédé selon la revendication 1 ou 2, **caractérisé en ce qu'**il génère :

- un ou plusieurs hydrocarbures, en particulier du méthane, du CO, un ou plusieurs alcools, de l'acide formique ou leurs mélanges, à partir de la conversion du $CO_2$,
- un ou plusieurs hydrocarbures, en particulier du méthane, un ou plusieurs alcools, de l'acide formique ou leurs mélanges, à partir de la conversion du CO.

**4.** Procédé selon l'une quelconque des revendications 1 à 3, **caractérisé en ce qu'**il génère du méthane.

**5.** Procédé selon l'une quelconque des revendications 1 à 4, **caractérisé en ce que** le procédé est réalisé en présence de dihydrogène.

**6.** Procédé selon la revendication 5, **caractérisé en ce que** le ratio molaire entre le $CO_2$ et/ou le CO et le dihydrogène est adapté de sorte à générer :

- un ou plusieurs hydrocarbures, en particulier du méthane, du CO, un ou plusieurs alcools, de l'acide formique ou leurs mélanges, à partir de la conversion du $CO_2$,
- un ou plusieurs hydrocarbures, en particulier du méthane, un ou plusieurs alcools, de l'acide formique ou leurs mélanges, à partir de la conversion du CO.

**7.** Procédé selon la revendication 5 ou 6, **caractérisé en ce que** le procédé est réalisé en présence de $CO_2$ et le ratio molaire $CO_2/H_2$ est de 1 : 4, de sorte à générer du méthane.

**8.** Procédé selon l'une quelconque des revendications 1 à 7, **caractérisé en ce que** la teneur massique en promoteur dans le système catalytique va de 0,1% à 20% en poids par rapport au poids du support, notamment de 0,5% à 15% en poids, de préférence va de 1% à 10% en poids.

**9.** Procédé selon l'une quelconque des revendications 1 à 8, **caractérisé en ce que** le support est un oxyde mixte de cérium et de zirconium.

**10.** Procédé selon la revendication 9, **caractérisé en ce que** le ratio molaire cérium/zirconium est compris dans une gamme allant de 90/10 à 40/60, préférablement de 80/20 à 50/50.

**11.** Procédé selon l'une quelconque des revendications 1 à 10, caractérisé en ce la teneur massique en nickel dans le système catalytique va de 3% à 30% en poids par rapport au poids du support, de préférence va de 7% à 17%.

**12.** Procédé selon l'une quelconque des revendications 1 à 11, **caractérisé en ce que** le support le nickel et le promoteur forment un mélange homogène.

**13.** Système catalytique comprenant :

- un support comprenant du cérium et/ou du zirconium,
- du nickel, et
- un promoteur choisi parmi le gadolinium et les mélanges gadolinium et yttrium.

**14.** Système catalytique selon la revendication 13, **caractérisé en ce que** le promoteur est le gadolinium.

**Patentansprüche**

**1.** Verfahren zur Umwandlung eines Gases, das $CO_2$ und/oder CO umfasst, in Gegenwart eines Katalysatorsystems und eines kalten Plasmas, vorzugsweise eines Plasmas, das durch dielektrische Barriereentladung (DBD) erzeugt wird, das Katalysatorsystem umfassend:

- ein Träger, der Cerium und/oder Zirkonium umfasst,
- Nickel und

- ein Vermittler, der unter Gadolinium und den Mischungen Gadolinium und Yttrium ausgewählt wird.

**2.** Verfahren nach Anspruch 1, **dadurch gekennzeichnet, dass** der Vermittler Gadolinium ist.

**3.** Verfahren nach Anspruch 1 oder 2, **dadurch gekennzeichnet, dass** es Folgendes erzeugt:

- einen oder mehrere Kohlenwasserstoffe, insbesondere Methan, CO, einen oder mehrere Alkohole, Ameisensäure oder Mischungen davon, aus der Umwandlung von $CO_2$,
- einen oder mehrere Kohlenwasserstoffe, insbesondere Methan, einen oder mehrere Alkohole, Ameisensäure oder Mischungen davon, aus der Umwandlung von CO.

**4.** Verfahren nach einem der Ansprüche 1 bis 3, **dadurch gekennzeichnet, dass** es Methan erzeugt.

**5.** Verfahren nach einem der Ansprüche 1 bis 4, **dadurch gekennzeichnet, dass** das Verfahren in Gegenwart von Diwasserstoff durchgeführt wird.

**6.** Verfahren nach Anspruch 5, **dadurch gekennzeichnet, dass** das Molverhältnis zwischen $CO_2$ und/oder CO und Diwasserstoff so angepasst ist, dass es Folgendes erzeugt:

- einen oder mehrere Kohlenwasserstoffe, insbesondere Methan, CO, einen oder mehrere Alkohole, Ameisensäure oder Mischungen davon, aus der Umwandlung von $CO_2$,
- einen oder mehrere Kohlenwasserstoffe, insbesondere Methan, einen oder mehrere Alkohole, Ameisensäure oder Mischungen davon, aus der Umwandlung von CO.

**7.** Verfahren nach Anspruch 5 oder 6, **dadurch gekennzeichnet, dass** das Verfahren in Gegenwart von $CO_2$ durchgeführt wird und das Molverhältnis $CO_2/H_2$ 1 beträgt: 4, so dass Methan entsteht.

**8.** Verfahren nach einem der Ansprüche 1 bis 7, **dadurch gekennzeichnet, dass** der Massengehalt des Vermittlers im Katalysatorsystem von 0,1 Gew.-% bis 20 Gew.-% bezogen auf das Gewicht des Trägers, insbesondere von 0,5 Gew.-% bis 15 Gew.-%, vorzugsweise von 1 Gew.-% bis 10 Gew.-% beträgt.

**9.** Verfahren nach einem der Ansprüche 1 bis 8, **dadurch gekennzeichnet, dass** der Träger ein Mischoxid aus Cerium und Zirkonium ist.

**10.** Verfahren nach Anspruch 9, **dadurch gekennzeichnet, dass** das Molverhältnis Cerium/Zirkonium in einem Bereich von 90/10 bis 40/60, vorzugsweise von 80/20 bis 50/50 liegt.

**11.** Verfahren nach einem der Ansprüche 1 bis 10, **dadurch gekennzeichnet, dass** der Massenanteil an Nickel im Katalysatorsystem bezogen auf das Gewicht des Trägers von 3 bis 30 Gew.-%, vorzugsweise von 7 bis 17 Gew.-%, beträgt.

**12.** Verfahren nach einem der Ansprüche 1 bis 11, **dadurch gekennzeichnet, dass** der Träger Nickel und der Vermittler ein homogenes Gemisch bilden.

**13.** Katalysatorsystem umfassend:

- ein Träger, der Cerium und/oder Zirkonium umfasst,
- Nickel und
- ein Vermittler, der unter Gadolinium und den Mischungen Gadolinium und Yttrium ausgewählt wird.

**14.** Katalysatorsystem nach Anspruch 13, **dadurch gekennzeichnet, dass** der Vermittler Gadolinium ist.

**Claims**

**1.** Process for converting a gas comprising $CO_2$ and/or CO in the presence of a catalytic system and a cold plasma, preferably a plasma generated by dielectric barrier discharge (DBD), said catalytic system comprising:

- a support comprising cerium and/or zirconium,
- nickel, and
- a promoter chosen from gadolinium and mixtures of gadolinium and yttrium.

2. Process according to claim 1, **characterised in that** it the promoter is gadolinium.

3. Process according to claim 1 or 2, **characterised in that** it generates:

- one or more hydrocarbons, in particular methane, CO, one or more alcohols, formic acid or mixtures thereof, from the conversion of the $CO_2$,
- one or more hydrocarbons, in particular methane, one or more alcohols, formic acid or mixtures thereof, from the conversion of the CO.

4. Process according to any of claims 1 to 3, **characterised in that** it generates methane.

5. Process according to any of claims 1 to 4, **characterised in that** the process is carried out in the presence of dihydrogen.

6. Process according to claim 5, **characterised in that** the molar ratio between the $CO_2$ and/or the CO and the dihydrogen is adapted so as to generate:

- one or more hydrocarbons, in particular methane, CO, one or more alcohols, formic acid or mixtures thereof, from the conversion of the $CO_2$,
- one or more hydrocarbons, in particular methane, one or more alcohols, formic acid or mixtures thereof, from the conversion of the CO.

7. Process according to claim 5 or 6, **characterised in that** the process is carried out in the presence of $CO_2$ and the $CO_2/H_2$ molar ratio is 1:4, so as to generate methane.

8. Process according to claim 1 or 7, **characterised in that** the mass concentration of promoter in the catalytic system ranges from 0.1% to 20% by weight relative to the weight of the support, in particular from 0.5% to 15% by weight, preferably ranges from 1% to 10% by weight.

9. Process according to claim 1 or 8, **characterised in that** the support is a mixed oxide of cerium and of zirconium.

10. Process according to claim 9, **characterised in that** the cerium/zirconium molar ratio is within a range going from 90/10 to 40/60, preferably from 80/20 to 50/50.

11. Process according to any one of claims 1 to 10, **characterised in that** the mass concentration of nickel in the catalytic system ranges from 3% to 30% by weight relative to the weight of the support, preferably ranges from 7% to 17%.

12. Process according to any one of claims 1 to 11, **characterised in that** the support, the nickel and the promoter form a homogenous mixture.

13. Catalytic system comprising:

- a support comprising cerium and/or zirconium,
- nickel, and
- a promoter chosen from gadolinium and mixtures of gadolinium and yttrium.

14. Catalytic system according to claim 13, **characterised in that** the promoter is gadolinium.

**Figure 1**

A)

B)

**Figure 2**

Figure 3

**EP 4 237 140 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2020128009 A

- EP 3050865 A1, Amouroux J

**Littérature non-brevet citée dans la description**

- **F. OCAMPO** ; **B. LOUIS** ; **A.-C. ROGER**. Methanation of carbon dioxide over nickelbased Ce0.72Zr0.28O2 mixed oxide catalysts prepared by sol-gel method. *Applied Catalysis A: General*, November 2009, vol. 369 (1-2), 90-96
- **M. NIZIO** ; **A. ALBARAZI** ; **S. CAVADIAS** ; **J. AMOUROUX** ; **M. E. GALVEZ** ; **P. DA COSTA**. Hybrid plasma-catalytic methanation of CO2 at low temperature over ceria zirconia supported Ni catalysts. *International Journal of Hydrogen Energy*, July 2016, vol. 41 (27), 11584-11592
- **J. AMOUROUX** ; **S. CAVADIAS**. Electrocatalytic reduction of carbon dioxide under plasma DBD process. *J. Phys. D: Appl. Phys.*, November 2017, vol. 50 (46), 465501
- **M. MIKHAIL et al.** Plasma-catalytic hybrid process for CO2 methanation: optimization of operation parameters. *Reac Kinet Mech Cat*, April 2019, vol. 126 (2), 629-643
- **M. MIKHAIL et al.** Electrocatalytic behaviour of CeZrOx-supported Ni catalysts in plasma assisted CO2 methanation. *Catal. Sci. Technol.*, 2020, vol. 10 (14), 4532-4543
- **M. NIZIO et al.** Low temperature hybrid plasma-catalytic methanation over Ni-Ce-Zr hydrotalcite-derived catalysts. *Catalysis Communications*, August 2016, vol. 83, 14-17

- **E. JWA** ; **S. B. LEE** ; **H. W. LEE** ; **Y. S. MOK**. Plasma-assisted catalytic methanation of CO and CO2 over Ni-zeolite catalysts. *Fuel Processing Technology*, April 2013, vol. 108, 89-93
- **M. BISET-PEIRÔ** ; **J. GUILERA** ; **T. ZHANG** ; **J. ARBIOL** ; **T. ANDREU**. On the role of ceria in Ni-Al2O3 catalyst for CO2 plasma methanation. *Applied Catalysis A: General*, April 2019, vol. 575, 223-229
- **M. C. BACARIZA et al.** DBD plasma-assisted CO2 methanation using zeolite-based catalysts: Structure composition-reactivity approach and effect of Ce as promoter. *Journal of CO2 Utilization*, July 2018, vol. 26, 202-211
- **T. C. MANLEY**. The Electric Characteristics of the Ozonator Discharge. *Trans. Electrochem. Soc.*, 1943, vol. 84 (1), 83
- **R. VALDIVIA-BARRIENTOS** ; **J. PACHECO-SOTELO** ; **M. PACHECO-PACHECO** ; **J. S. BENÍTEZ-READ** ; **R. LÔPEZ-CALLEJAS**. Analysis and electrical modelling of a cylindrical DBD configuration at different operating frequencies. *Plasma Sources Sci. Technol.*, May 2006, vol. 15 (2), 237-245